# EUROPEAN PATENT SPECIFICATION

(11) **EP 2 070 533 B1**
(45) Date of publication and mention of the grant of the patent: **07.05.2014**
(21) Application number: 07380346.2
(22) Date of filing: 11.12.2007
(51) Int. Cl.: A61K 31/365, A61K 9/00, A61P 17/00, A61P 17/06

(54) **Use of a compound derived from P-hydroxyphenyl propionic acid for the treatment of psoriasis**
Verwendung einer aus P-Hydroxyphenyl-Propionsäure entwickelten Verbindung zur Behandlung von Psoriasis
Utilisation d'un composant dérivé de l'acide propionique P-hydroxyphényl pour le traitement de la psoriasis

(43) Date of publication of application: 17.06.2009
(73) Proprietor: Apoteknos Para La Piel, s.l., Madrid (ES)
(72) Inventor: Pivel Ranieri, Juan Pablo, 28760 Tres Cantos (Madrid) (ES); Ferrer Cuesta, Juan Manuel, 28760 Tres Cantos (Madrid) (ES); Martinez Galán, Fernando, 31008 Pamplona (Navarra) (ES); Irache, Juan Manuel, 31008 Pamplona (Navarra) (ES); Novella Robisco, Jose Luis, 28871 Alcalá de Henares (Madrid) (ES); Perez Rueda, Juan Jose, 28871 Alcalá de Henares (Madrid) (ES); Maria Martin, Maria Paz, 28871 Alcalá de Henares (Madrid) (ES); Builla Gomez, Julio Álvarez, 28871 Alcalá de Henares (Madrid) (ES); Bermejo, Jaime, 38206 La Laguna (Tenerife) (ES); Vidal Vanaclocha, Fernando, 48940 Leiona Vizcaya (ES)
(74) Representative: Garcia-Cabrerizo y del Santo, Pedro Maria

(56) References cited:
- EP-B- 1 284 263
- BRAEUTIGAM M ET AL: "EFFECTS OF CALCIPOTRIOL MC 903 AND CALCITRIOL AFTER TOPICAL APPLICATION ON THE SKIN OF HAIRLESS RATS MUCH LOWER EFFECT OF CALCIPOTRIOL ON SYSTEMIC CALCIUM HOMEOSTASIS", SKIN PHARMACOLOGY, vol. 5, no. 2, 1992, pages 87-92, ISSN: 1011-0283
- ZENZ R ET AL: "Psoriasis-like skin disease and arthritis caused by inducible epidermal deletion of Jun proteins", NATURE 20050915 NATURE PUBLISHING GROUP GB, vol. 437, no. 7057, 15 September 2005 (2005-09-15), pages 369-375, DOI: DOI:10.1038/NATURE03963

## Description

The present invention relates to the use of compound A (derived from P-hydroxyphenyl propionic acid): 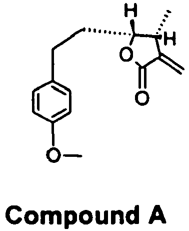 for the treatment of psoriasis.

### BACKGROUND

Psoriatic skin is characterised by the hyperproliferation of keratinocytes, inflammation, angiogenisis and dilation of the blood vessels. There are different types of treatments on the market to prevent or reduce this type of skin disorder that is prevalent in 2% of the world's population, such as the use of topical creams with certain compositions such as those disclosed in the prior art ES2214105, ES2231007, ES2188426 and ES2186586, or specific products such as derivatives of vitamin D and retinoic acid, and certain corticosteroids. There are also many publications that show the great social impact of this disorder *(*mechanism of psoriasis" Frank O. Nestle, Curdin Conrad, Drug Discovery Today: Disease Mechanisms Vol. I No. 3 2004*)* and the importance of finding compounds to help in its treatment and enable its elimination *("*Dermatology Online Journal" Volume 6 No. 1 Steven Feldman, M.D., PhD*).* Most products for psoriasis that are known at present are derived from the use of products initially developed for other pathologies such as rheumatoid arthritis, inflammatory bowel disease, etc. and they are very aggressive treatments, or strong immunosuppressants such as cyclosporine, rapamycin and methotrexate, or very expensive treatments such as anti-TNF monoclonal antibodies, which cannot be used as chronic therapies and have serious side effects.

Calcitriol (1-alpha,25-dihydroxycholecalciferol) is the active form of vitamin D that is found in the body (vitamin D₃) and its use is known for the treatment of psoriasis, as is disclosed in various publications and patents such as *"*Calcitriol ointment and clobetasol propionate cream: a new regimen for the treatment of plaque psoriasis" European Journal of Dermatology, Vol. II, No. 3, May 2003 (2003-05) pp.261-265*,* WO2006008354 or US4610978.

The document EP1284263, which is regarded as a closest prior art, discloses derivatives of p-hydroxyphenyl propionic acid, which have antiproliferative activity.

In an attempt to find new compositions that make it possible to treat this disorder, it has surprisingly been discovered that compound A has the following important characteristics:
1) Safe and effective compound that does not present the side effects of vitamin D derivatives.
2) Low permeability, which makes it possible to define the product as topical and enables the mutagenesis test (Ames test) to be performed without hepatic metabolisation, as the product is not absorbed topically.
3) It is an active topical product that does not cause toxic effects and is therefore a safe product.
4) Its antipsoriatic activity is similar to that of calcitriol.

### DESCRIPTION OF THE INVENTION

Various studies have been carried out to show the benefits of using compound A: 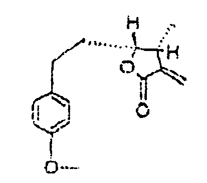 to treat psoriasis.

### 1. Permeability study

Skin from pig ears was used as the experimental system to which a hydroalcoholic solution of compound A 6.36x10⁻² mg/ml and 6.36x10⁻³ mg/ml was applied, the reference product being a control hydroalcoholic solution (without compound A). For this study 4 different hydroalcoholic solutions were prepared. The first included 6.36 µg/ml of compound A, the second included 63.6 µg/ml, and the third and fourth were control solutions without compound A.

All the preparations were tested on the pig ear skin experimental system. Samples were taken in the diffuser compartment at 6 different times (0, 1, 2, 6, 12 and 24 hours). All the samples (recipients) were quantified by HPLC.

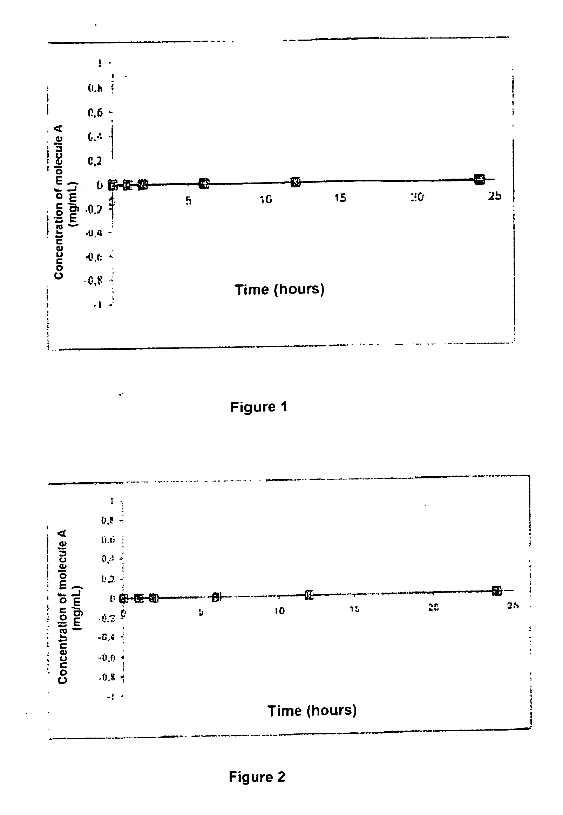

Figures 1 and 2 show the amount of compound A collected in the recipient:

Figure 1. Concentration of compound A in the recipient of the Franz cell for a sample with a concentration of 63.6 µg/ml of A.

Figure 2. Concentration of compound A in the recipient of the Franz cell for a sample with a concentration of 6.36 µg/ml of A.

As can be observed, in every case the amount of compound A dissolved in the recipient was lower that 0.01 mg/ml (quantification limit of the HPLC technique). Moreover, in no case could any sign be observed that would make it possible to predict the passage of a minimum fraction of molecule A. Therefore, is can be stated that in the conditions used in the study there is no transdermal flow of compound A through the skin of the pig ear.

In conclusion, it can be said that molecule A, in the formulated hydroalcoholic vehicle, does not cross the pig's skin in the conditions tested, at least in a sufficient amount to be detected and quantified using the proposed analytical technique.

### 2. Ames test

The mutagenic activity of compound A was evaluated using the Ames test without external metabolisation. The Ames test is based on the use of bacterial strains to detect mutations in the DNA in vitro using bacterial strains that make it possible to detect types of damage to the genome. As the product is defined as topical, since it is not permeable as is shown in the previous test, the mutagenisis test (Ames test) is performed without hepatic metabolisation, as the topical product is not absorbed.

Bacteria of 4 strains of *Salmonella typhimurium* that are internationally recommended for this test, TA1535, TA1537, TA98 and TA100, were used. The test was carried out according to highly standardised protocols. Three concentrations of compound A were tested, being seeded on 3 plates for each concentration in a single experiment. The test was carried out without external metabolic activation. The plates were incubated at 37°C and the reverting colonies were counted after 48 hours.

The results are as follows:

**Table 1. Salmonella typhimurium TA98**

| | Coloniesper plate | | | | Mean | SD | Mutagenic Ind, |
|---|---|---|---|---|---|---|---|
| | 1 | 2 | 3 | 4 | | | |
| H-/B- | 0 | 0 | 0 | 0 | 0 | 0 | 0 |
| H+/B+ | 8 | 11 | 12 | 7 | 9.5 | 2.38047614 | 1 |
| H+/B+ DMSO | 10 | 13 | 10 | 10 | 10.75 | 1.5 | 1.131578947 |
| H+/B+ Comp, A 10 µg/ml | 9 | 6 | 12 | 14 | 10.25 | 3.5 | 1.078947368 |
| H+/B+ Comp, A 100 µg/ml | 15 | 12 | 8 | 12 | 11.75 | 2.87228132 | 1.236842105 |
| H+/B+ Comp, A 1 mg/ml | 8 | 4 | 6 | 17 | 8.75 | 5.73730483 | 0.921052632 |
| H+/B+ 4-Nitro-0-Phe... (2.5 µg/plate) | 29 | 29 | 17 | 22 | 24.25 | 5.85234996 | 2.552631579 |

**Table 2. Salmonella typhimurium TA100**

| | Colonies per plate | | | | Mean | SD | Mutagenic Ind, |
|---|---|---|---|---|---|---|---|
| | 1 | 2 | 3 | 4 | | | |
| H-/B- | 0 | 0 | 0 | 0 | 0 | 0 | 0 |
| H+/B+ | 66 | 55 | 35 | 38 | 48.5 | 14.6173413 | 1 |
| H+/B+ DMSO | 31 | 28 | 52 | 43 | 38.75 | 10.7819293 | 0.798969072 |
| H+/B+ Comp, A 10 µg/ml | 38 | 41 | 44 | 54 | 44.25 | 6.94622199 | 0.912371134 |
| H+/B+ Comp, A 100 µg/ml | 45 | 35 | 31 | 51 | 40.5 | 9.14694849 | 0.835051546 |
| H+/B+ Comp, A 1 mg/ml | 52 | 37 | 38 | 40 | 44 | 7.61577311 | 0.907216495 |
| H+/B+ 4-Nitro-0-Phe... (2.5 µg/plate) | 94 | 72 | 74 | 87 | 81.75 | 10.5316982 | 1.68556701 |

**Table 3. Salmonella typhimurium TA1535**

| | Colonies per plate | | | | Mean | SD | Mutagenic Ind, |
|---|---|---|---|---|---|---|---|
| | 1 | 2 | 3 | 4 | | | |
| H-/B- | 0 | 0 | 0 | 0 | 0 | 0 | 0 |
| H+/B+ | 0 | 4 | 3 | 1 | 2 | 1.82574186 | 1 |
| H+/B+ DMSO | 5 | 1 | 1 | 0 | 7.75 | 2.21735578 | 0.875 |
| H+/B+ Comp, A 10 µg/ml | 1 | 2 | 1 | 0 | 1 | 0.81649658 | 0.5 |
| H+/B+ Comp, A 100 µg/ml | 1 | 2 | 1 | 0 | 1 | 0.81649658 | 0.5 |
| H+/B+ Comp, A 1 mg/ml | 1 | 0 | 2 | 1 | 1 | 0.81649658 | 0.5 |
| H+/B+ 4-Nitro-0-Phe... (2.5 µg/plate) | 70 | 102 | 103 | 76 | 87.75 | 172119145 | 43.875 |

**Table 4. Salmonella typhimurium TA1537**

| | Colonies per plate | | | | Mean | SD | Mutagenic Ind, |
|---|---|---|---|---|---|---|---|
| | 1 | 2 | 3 | 4 | | | |
| H-/B- | 0 | 0 | 0 | 0 | 0 | 0 | 0 |
| H+/B+ | 0 | 1 | 0 | 1 | 0.5 | 0.57735027 | 1 |
| H+/B+ DMSO | 1 | 1 | 1 | 1 | 1 | 0 | 2 |
| H+/B+ Comp, A 10 µg/ml | 0 | 1 | 0 | 0 | 0.25 | 0.5 | 0.5 |
| H+/B+ Comp, A 100 µg/ml | 0 | 1 | 0 | 2 | 0.75 | 0.95742711 | 1.5 |
| H+/B+ Comp, A 1 mg/ml | 0 | 0 | 0 | 0 | 0 | 0 | 0 |
| H+/B+ 4-Nitro-0-Phe... (2.5 µg/plate) | 74 | 135 | 46 | 56 | 77.75 | 39.8862967 | 155.5 |

As can be observed, there was no increase in the dose according to the reversion frequency per colony for any of the *Salmonella* strains that were tested, which is the mutagenicity indicator for this test. It can therefore be concluded that the results were negative in all the tested strains of *Salmonella typhimurium* (TA1535, TA1537, TA98 and TA100), and compound A does not induce mutagenic effects at the concentrations evaluated.

### 3. Evaluation of antipsoriatic activity

The activity of compound A on cell hyperproliferation in hairless rats was evaluated using the model of desquamation in hairless rats described in the publication: Bräutigam, M., Hübner, H., Rach, P., Thieroff-Ekerdi, R. "Effects of Calcipotriol (MC 903) and Calcitriol after Topical Application on the Skin of Hairless Rats", Skin Pharmacol 1992; 5:87-92.

To do this Calcitriol was used as the reference product at 0.01% and compound A was used as the test product at 1% and 0.1 % (100 µg/cm² and 10 µg/cm², respectively). A Control Group was included, administered only with the vehicle (absolute ethanol).

The animals were randomly distributed into the following experimental groups:

**Table 5: Experimental Group Distribution**

| **Treatment** | **Dose** | **n** | **Group** | **Volume of administration** | **Route of administration** |
|---|---|---|---|---|---|
| Control | | | A | | Topical |
| Calcitriol 0.01% | 1 µg/cm² | 6 | B | 100 µl/10cm² | |
| Comp. A 1% | 100 µg/cm² | 6 | C | 100 µl/10cm² | |
| Comp. A 0.11% | 10 µg/cm² | 6 | D | 100 µl/10cm² | |

The treatments were administered topically, once a day for 10 days (100 µg/side/animal). The animals were weighed on a daily basis throughout the study and the grade of erythema was evaluated (0 = Absence of erythema, 1 = Low grade erythema, 3 = High grade erythema). On the last day of the study, day 11, the amount of corneous material extracted from the epidermis of the animals was evaluated.

Tables 6, 7 and 8 show the overall values corresponding to the amount of corneous material extracted, the mean evaluation of erythema and the body weight of the animals, respectively:

**Table 6: Overall evaluation of the amount of corneous material extracted**

| **Group** | **Treatment** | **n** | **Route of administration** | **Volume of administration** | **Total sum of the right and left flanks (mg)** | | | | | |
|---|---|---|---|---|---|---|---|---|---|---|
| | | | | | Portal No, 1 | Portal No, 2 | Portal No, 3 | Portal No, 4 | Portal No, 5 | Portal No, 6 |
| A | Control | 6 | Topically | 0.2 mL/animal | 3.3 ±0.21 | 2.4 ±0.64 | 1.0 ±0.12 | 0.8 ±0.13 | 0.83 ±0.23 | 8.2 ±0.81 |
| B | Calcitriol | 6 | Topically | 0.2 mL/animal | 13.5 ±4.89 | 7.7 ±1.43 | 5.9 ±0.94 | 4.5 ±0.60 | 3.7 ±0.35 | 21.7 ±2.07 |
| | 0.01% | | | | | | | | | |
| C | Compound A | 6 | Topically | 0.2 mL/animal | 3.1 ±0.75 | 2.7 ±0.23 | 2.0 ±0.26 | 1.4 ±0.169 | 1.4 ±0.23 | 12.6 ±0.76 |
| | 1% | | | | | | | | | |
| D | Compound A | 6 | Topically | 0.2 mL/animal | 3.1 ±0.17 | 1.7 ±0.08 | 1.1 ±0.14 | 1.1 ±0.22 | 1.1 ±0.28 | 8.1 ±0.47 |
| | 0.1% | | | | | | | | | |

**Table 7: Mean evaluation of erythema**

| **Group** | **Treatment** | **n** | **Route of administration** | **Volume of administration** | **Assessment of erythema** | | | | | | | | | | |
|---|---|---|---|---|---|---|---|---|---|---|---|---|---|---|---|
| | | | | | Baseline | Day 2 | Day 3 | Day 4 | Day 5 | Day 6 | Dau 7 | Day 8 | Day 9 | Day 10 | Day 11 |
| A | Control | 6 | Topically | 0.2 mL/animal | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 |
| B | Calcitriol | 6 | Topically | 0.2 mL/animal | 0.0 ±0.00 | 0.0 ±0.00 | 0.0 ±0.00 | 0.0 ±0.00 | 0.0 ±0.00 | 0.0 ±0.00 | 0.0 ±0.00 | 1.0 ±0.00 | 1.5 ±0.22 | 2.0 ±0.20 | 2.0 ±0.00 |
| | 0.01% | | | | | | | | | | | | | | |
| C | Compound A | 6 | Topically | 0.2 mL/animal | 0.0 ±0.00 | 1.0 ±0.00 | 1.0 ±0.00 | 1.2 ±0.11 | 1.3 ±0.17 | 1.5 ±0.22 | 1.5 ±0.22 | 1.2 ±0.40 | 0.9 ±0.15 | 0.9 ±0.15 | 1.2 ±0.17 |
| | 1% | | | | | | | | | | | | | | |
| D | Compound A | 6 | Topically | 0.2 mL/animal | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 |
| | 0.1% | | | | | | | | | | | | | | |

**Table 8: Overall evaluation of body weight**

| **Group** | **Treatment** | **n** | **Route of administration** | **Volume of administration** | **Body weight (g)** | | | | | | | | | | |
|---|---|---|---|---|---|---|---|---|---|---|---|---|---|---|---|
| | | | | | Baseline | Day 2 | Day 3 | Day 4 | Day 5 | Day 6 | Dau 7 | Day 8 | Day 9 | Day 10 | Day 11 |
| A | Control | 6 | Topically | 0.2 mL/animal | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 |
| B | Calcitriol | 6 | Topically | 0.2 mL/animal | 0.0 ±0.00 | 0.0 ±0.00 | 0.0 ±0.00 | 0.0 ±0.00 | 0.0 ±0.00 | 0.0 ±0.00 | 0.0 ±0.00 | 1.0 ±0.00 | 1.5 ±0.22 | 2.0 ±0.20 | 2.0 ±0.00 |
| | 0.01% | | | | | | | | | | | | | | |
| C | Compound A | 6 | Topically | 0.2 mL/animal | 0.0 ±0.00 | 1.0 ±0.00 | 1.0 ±0.00 | 1.2 ±0.11 | 1.3 ±0.17 | 1.5 ±0.22 | 1.5 ±022 | 1.2 ±0.40 | 0.9 ±0.15 | 0.9 ±0.15 | 1.2 ±0.17 |
| | 1% | | | | | | | | | | | | | | |
| D | Compound A | 6 | Topically | 0.2 mL/animal | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 | 0.0 ±0.00 |
| | 0.1% | | | | | | | | | | | | | | |

From the results obtained, it can be concluded that after topical application of 0.2 ml/animal of the reference product Calcitriol 0.01%, it was observed that the amount of corneous material extracted was clearly greater than that extracted in the other experimental groups. However, in this treatment group, at the specified dose, clear signs of toxicity were detected. This increase in corneous material that was susceptible to desquamation is described as a possible consequence of a terminal differentiation of keratinocytes in the epidermis.

After the group treated with the reference substance, the group that was administered compound A at 1% was the group from which the greatest amount of corneous material was extracted, presenting statistically significant differences in relation to the control group and the group treated with compound A at 0.1% (Student Newman Keuls, p<0.01), possibly due to an effect on the epidermis similar to that described for Calcitriol. Despite observing that the amount of corneous material extracted in this group was lower than that of the reference group, none of the animals presented signs of toxicity, which means that it is considered a safer dose than that of the Calcitriol 0.01 % treatment.

The control group and the group treated with compound A at 0.1% presented values that were lower than those observed in the other groups, and it was considered that it had no effect on the animals' skin.

Three of the animals in the Calcitriol 0.01% group died before the end of the study, possibly due to toxic effects resulting from the repeated administration of the product.

From the studies that were carried out it can be concluded that compound A has clear beneficial effects for the treatment of psoriasis and can therefore be used for the treatment of said disorder in pharmaceutical compositions for topical use that can be administered in the form of a cream, gel or any other normal pharmaceutical form.

## Claims

1. Use of the compound with the chemical formula 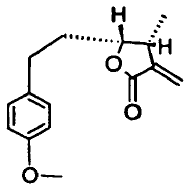 for the preparation of topical administration compositions for the treatment of skin disorders in humans.

2. Use according to claim 1, wherein the skin disorder is psoriasis.

3. Pharmaceutical composition suitable for topical use on the skin, **characterised in that** it contains, in a proportion of between 0.1% and 5% w/w, the active compound with the chemical formula

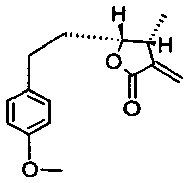

4. Pharmaceutical composition according to claim 3 that is administered in the form of gel or cream.

## Patentansprüche

1. Verwendung der Verbindung mit der chemischen Formel 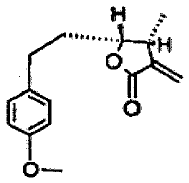 für die Herstellung von Zusammensetzungen zur topischen Verabreichung zur Behandlung von Hauterkrankungen bei Menschen.

2. Verwendung nach Anspruch 1, wobei es sich bei der Hauterkrankung um Psoriasis handelt.

3. Pharmazeutische Zusammensetzung, welche für die topische Anwendung auf der Haut geeignet ist, **dadurch gekennzeichnet, dass** sie in einem Verhältnis von 0,1 % bis 0,5 % w/w den Wirkstoff mit der chemischen Formel 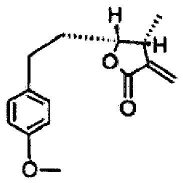 enthält.

4. Pharmazeutische Zusammensetzung nach Anspruch 3, welche in Form von Gel oder Creme verabreicht wird.

## Revendications

1. Utilisation du composé dont la formule chimique est : 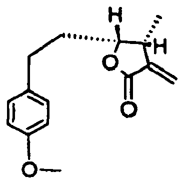 pour la préparation de compositions d'administration topique pour le traitement des affections cutanées chez l'homme.

2. Utilisation selon la revendication 1, dans laquelle l'affection cutanée est le psoriasis.

3. Composition pharmaceutique adéquate pour une utilisation topique sur la peau **caractérisée en ce qu'**elle contient, dans une proportion comprise entre 0,1 % et 5 % poids/poids, le composé actif dont la formule chimique est :

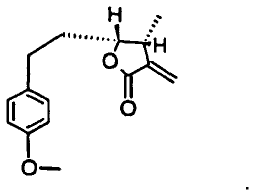

4. Composition pharmaceutique selon la revendication 3 qui est administrée sous forme de gel ou de crème.
